# EUROPÄISCHE PATENTANMELDUNG

(11) **EP 0 422 369 A2**
(43) Veröffentlichungstag der Anmeldung: **17.04.1991**
(21) Anmeldenummer: 90116317.0
(22) Anmeldetag: 25.08.1990
(51) Int. Cl.: C07D 249/12, C07D 401/12, C07D 413/12, C07D 417/12, A01N 47/38, A01N 43/653, A01N 43/72

(54) **Substituierte Carbamoyltriazole**

(30) Priorität: 07.09.1989 DE 3929673
(71) Anmelder: BAYER AG, 51368 Leverkusen (DE)
(72) Erfinder: Jelich, Klaus, Dr., D-5600 Wuppertal 1 (DE); Schmidt, Robert R., Dr., D-5060 Bergisch Gladbach 2 (DE); Santel, Hans-Joachim, Dr., D-5090 Leverkusen 1 (DE); Lürssen, Klaus, Dr., D-5060 Bergisch Gladbach 2 (DE)

(57) **Zusammenfassung**

Neue substituierte Carbamoyltriazole, der allgemeinen Formel (I), 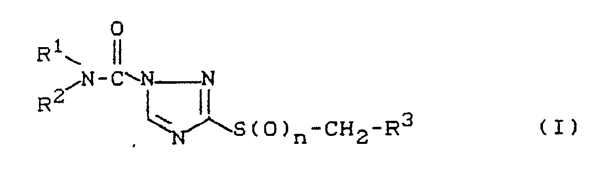
Verfahren zu ihrer Herstellung und ihre Verwendung als Herbizide.

## Beschreibung

Die Erfindung betrifft neue substituierte Carbamoyltriazole, Verfahren zu ihrer Herstellung und ihre Verwendung als Herbizide.

Es ist bekannt, daß bestimmte substituierte Carbamoyltriazole als Herbizide eingesetzt werden können (vgl. Pestic. Sci. 6 (1975), 665 - 673; DE-OS 2 132 618; DE-OS 2 264 159; US-P 4 280 831; EP-A 140194; JP-A 59-039 880; JP-A 61-178 980). Die Wirkung dieser Verbindungen ist jedoch nicht in allen Belangen zufriedenstellend.

Es wurden nun die neuen substituierten Carbamoyltriazole der allgemeinen Formel (I) 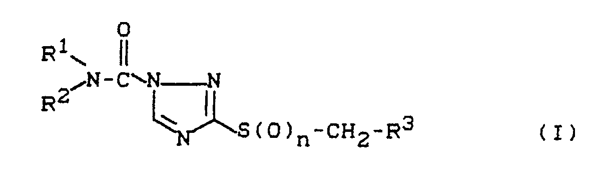 in welcher
R¹ und R² gleich oder verschieden sind und unabhängig voneinander jeweils für geradkettiges oder verzweigtes Alkyl mit 1 bis 6 Kohlenstoffatomen stehen,
R³ für 4-Chlor-phenyl, für Cyanophenyl, für Nitrophenyl, Methyl-nitrophenyl oder für einen fünfgliedrigen heteroaromatischen Cyclus steht, welcher ein oder zwei Stickstoffatome und zusätzlich ein Sauerstoff- oder Schwefel-atom als Heteroatome enthält und welcher gegebenenfalls einfach oder zweifach, gleich oder verschieden durch Halogen, Cyano, Nitro, Dimethylaminocarbonyl, Diethylaminocarbonyl oder durch jeweils geradkettiges oder verzweigtes Alkyl, Alkoxy, Alkylthio, Alkylsulfinyl oder Alkylsulfonyl mit jeweils 1 bis 6 Kohlenstoffatomen oder durch Phenyl substituiert ist, wobei das Phenyl gegebenenfalls einfach bis dreifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ weiterhin für einen sechsgliedrigen heteroaromatischen Cyclus steht, welcher ein oder zwei Stickstoffatome enthält und welcher gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ ferner für Benzoxazolyl oder Benzthia zolyl steht, welche jeweils gegebenenfalls einfach oder zweifach, gleich oder verschieden durch Halogen, Cyano, Nitro oder durch jeweils geradkettiges oder verzveigtes Alkyl oder Alkoxy mit jeweils 1 bis 4 Kohlenstoffatomen substituiert sind, und
n für die Zahlen 0, 1 oder 2 steht,
gefunden.

Weiter wurde gefunden, daß man die neuen Verbindungen der Formel (I) erhält, wenn man

(a) substituierte Triazole der allgemeinen Formel (II) 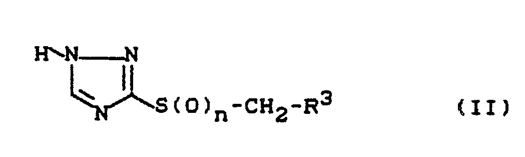 in welcher
R³ und n die oben angegebenen Bedeutungen haben, oder Tautomere zu den Verbindungen der Formel (II) mit Carbamidsäurechloriden der allgemeinen Formel (III) 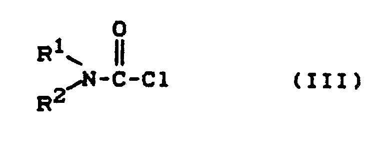 in welcher
R¹ und R² die oben angegebene Bedeutung haben, gegebenenfalls in Gegenwart eines Säureakzeptors und gegebenenfalls in Gegenwart eines Verdünnungsmittels umsetzt, oder wenn man
(b) substituierte Carbamoyltriazole der allgemeinen Formel (Ia) 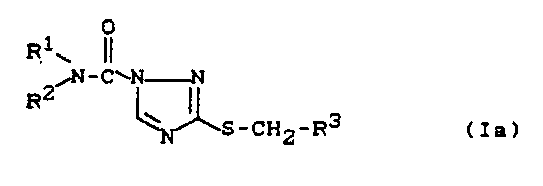 in welcher
R¹, R² und R³ die oben angegebene Bedeutung haben,
mit Oxidationsmitteln, gegebenenfalls in Gegenwart von Verdünnungsmitteln umsetzt.

Schließlich wurde gefunden, daß die neuen substituierten Carbamoyltriazole der allgemeinen Formel (I) interessante herbizide Eigenschaften aufweisen.

Die erfindungsgemäßen substituierten Carbamoyltriazole sind durch die Formel (I) allgemein definiert. Bevorzugt sind Verbindungen der Formel (I), bei welchen
R¹ und R² gleich oder verschieden sind und unabhängig voneinander jeweils für geradkettiges oder verzweigtes Alkyl mit 1 bis 4 Kohlenstoffatomen, ausgenommen tert-Butyl, stehen,
R³ für 4-Chlor-phenyl, für Cyanophenyl, für Nitrophenyl, Methyl-nitrophenyl oder für einen fünfgliedrigen heteroaromatischen Cyclus aus der Reihe Oxazolyl, Isoxazolyl, Thiazolyl, Isothiazolyl, Oxadiazolyl, Thiadiazolyl steht, welcher gegebenenfalls einfach oder zweifach, gleich oder verschieden durch Fluor, Chlor, Brom, Dimethylaminocarbonyl oder durch jeweils geradkettiges oder verzweigtes Alkyl, Alkoxy, Alkylthio, Alkylsulfinyl oder Alkylsulfonyl mit jeweils 1 bis 4 Kohlenstoffatomen substituiert ist, oder welcher durch Phenyl substituiert ist, wobei das Phenyl gegebenenfalls einfach oder zweifach, gleich oder verschieden durch Fluor, Chlor, Brom, Methyl, Ethyl, Methoxy oder Ethoxy substituiert ist, oder R³ weiterhin für Pyridyl oder Pyrimidyl steht, welche gegebenenfalls einfach oder zweifach, gleich oder verschieden durch Fluor, Chlor, Brom, Methyl, Ethyl, Methoxy oder Ethoxy substituiert sind, oder R³ ferner für Benzoxazolyl oder Benzthiazolyl steht, welche jeweils gegebenenfalls einfach oder zweifach, gleich oder verschieden durch Fluor, Chlor, Brom, Methyl, Ethyl, Methoxy oder Ethoxy substituiert sind, und
n für die Zahlen 0, 1 oder 2 steht.

Besonders bevorzugt sind Verbindungen der Formel (I), bei welchen
R¹ für Methyl, Ethyl oder n-Propyl steht,
R² für Methyl, Ethyl, n-Propyl, Isopropyl, n-Butyl oder Isobutyl steht,
R³ für 4-Chlor-phenyl, für Cyanophenyl, für Nitrophenyl, für Isoxazolyl, welches gegebenenfalls durch Chlor, Methyl, Ethyl, Propyl, Isopropyl, Butyl, Isobutyl, sec-Butyl, tert-Butyl oder Dimethylaminocarbonyl substituiert ist, für Thiazolyl, welches gegebenenfalls durch Chlor, Methyl oder Phenyl substituiert ist, für Oxadiazolyl, welches gegebenenfalls durch Methyl, Ethyl, Propyl, Isopropyl, Butyl, Isobutyl, sec-Butyl, tert-Butyl, Phenyl, Chlorphenyl oder Dichlorphenyl substituiert ist, für Pyridyl, welches gegebenenfalls durch Chlor, Methyl oder Methoxy substituiert ist, oder für Benzoxazolyl, welches gegebenenfalls durch Chlor substituiert ist, steht, und
n für die Zahlen 0, 1 oder 2 steht.

Verwendet man beispielsweise 3-(4-Cyano-benzylthio)-1H-1,2,4-triazol und N,N-Dimethyl-carbamidsäurechlorid als Ausgangsstoffe, so läßt sich der Reaktionsablauf beim erfindungsgemäßen Verfahren (a) durch das folgende Formelschema darstellen:

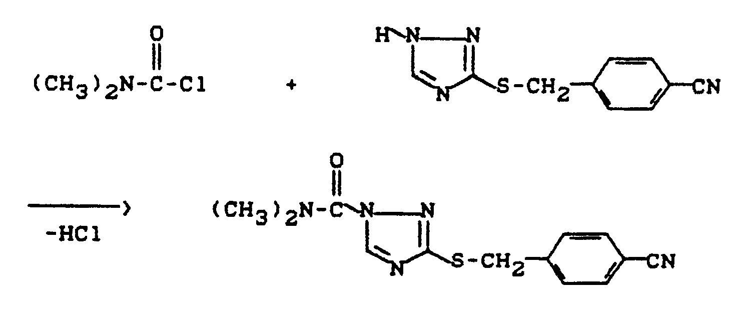

Verwendet man beispielsweise 1-(Dimethylaminocarbonyl)3-(pyridin-2-yl-methylthio)-1H-1,2,4-triazol und Hydrogenperoxid als Ausgangsstoffe so läßt sich der Reaktionsablauf beim erfindungsgemäßen Verfahren (b) durch das folgende Formelschema darstellen:

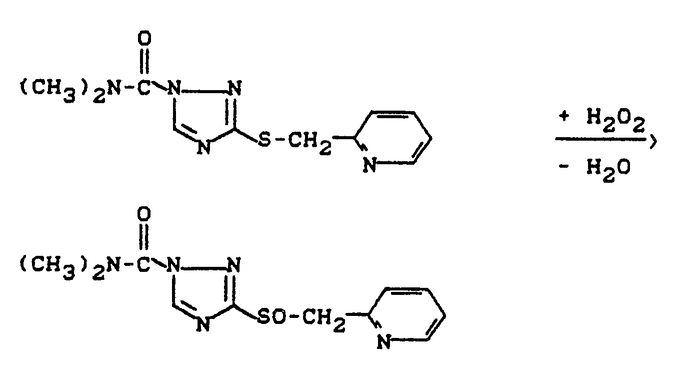

Die beim erfindungsgemäßen Verfahren (a) zur Herstellung von Verbindungen der Formel (I) als Ausgangsstoffe zu verwendenden substituierten Triazole sind durch die Formel (II) allgemein definiert.

In Formel (II) haben R³ und n vorzugsweise bzw. insbe sondere diejenigen Bedeutungen, die bereits oben im Zusammenhang mit der Beschreibung der erfindungsgemäßen Verbindungen der Formel (I) vorzugsweise bzw. als insbesondere bevorzugt für n und R³ angegeben wurden.

Die Ausgangsstoffe der Formel (II) sind noch nicht aus der Literatur bekannt.

Man erhält die neuen substituierten Triazole der Formel (II), wenn man 3-Mercapto-1,2,4-triazol der Formel (IVa) bzw. (IVb) 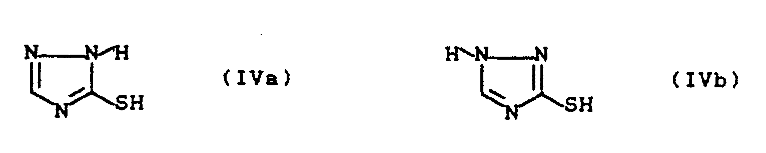 mit Alkylierungsmitteln der allgemeinen Formel (V)
X-CH₂-R³ (V)
in welcher
R³ die oben angegebene Bedeutung hat und
X für Halogen steht,
gegebenenfalls in Gegenwart eines Säureakzeptors wie z.B. Natriummethanolat, Kaliumcarbonat oder Pyridin, und gegebenenfalls in Gegenwart eines Verdünnungsmittels, wie z.B. Methanol, Acetonitril oder Dimethylformamid, bei Temperaturen zwischen 0°C und 100°C umsetzt und nach üblichen Methoden aufarbeitet (vgl. die Herstellungsbeispiele) sowie gegebenenfalls die so erhaltenen Verbindungen der Formel (II), bei denen n für Null steht, wie für das erfindungsgemäße Verfahren (b) beschrieben oxidiert.

Die benötigten Vorprodukte der Formeln (IVa)/(IVb) und (V) sind bekannte organische Chemikalien.

Die beim erfindungsgemäßen Verfahren (a) weiter als Ausgangsstoffe zu verwendenden Carbamidsäurechloride sind durch die Formel (III) allgemein definiert.

In Formel (III) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Ausgangsstoffe der Formel (III) sind bekannte organische Chemikalien.

Die beim erfindungsgemäßen Verfahren (b) benötigten Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,5-Diazabicyclo-[5,4,0]-undec-7-en (DBU) und 1,4-Diazabicyclo-[2,2,2]-octan (DABCO).

Soweit flüssige Säureakzeptoren, wie z.B. Pyridin, verwendet werden, können sie im Überschuß eingesetzt auch als Verdünnungsmittel dienen.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungs gemäßen Verfahren (a) jeweils nach üblichen Methoden.

Das erfindungsgemäße Verfahren (b) wird unter Einsatz eines Oxidationsmittels durchgeführt. Es können die üblichen zur Oxidation von Sulfiden zu Sulfoxiden oder Sulfonen verwendbaren Oxidationsmittel verwendet werden. Hierzu gehören beispielsweise Hydrogenperoxid, Perameisensäure, Peressigsäure, Perbenzoesäure und 3-Chlor-perbenzoesäure.

Das erfindungsgemäße Verfahren (b) wird vorzugsweise unter Verwendung von Verdünnungsmitteln durchgeführt. Hierzu gehören organische Losungsmittel wie Methylenchlorid, Chloroform, Ameisensäure, Essigsäure und Propionsäure, ferner auch Wasser.

Die Reaktionstemperaturen können bei dem erfindungsgemäßen Verfahren (b) in einem größeren Bereich variiert werden. Im allgemeinen arbeitet man bei Temperaturen zwischen -30°C und +100°C, vorzugsweise bei Temperaturen zwischen -10°C und +80°C.

Verfahren (b) wird im allgemeinen bei Normaldruck durchgeführt.

Zur Durchführung des erfindungsgemäßen Verfahrens (b) setzt man auf 1 Mol einer Verbindung der Formel (Ia) im allgemeinen 0,9 bis 1,5 Moläquivalente, vorzugsweise 1 bis 1,2 Moläquivalente eines Oxidationsmittels (zur Herstellung von Sulfoxiden) bzw. 1,9 bis 2,8 Moläquivalente, vorzugsweise 2 bis 2,5 Moläquivalente eines Oxidationsmittels (zur Herstellung von Sulfonen) ein.

Die Reaktionskomponenten werden im allgemeinen bei Raumtemperatur oder unter Kühlen zusammengegeben und das Reaktionsgemisch wird bei der jeweils erforderlichen Temperatur bis zum Ende der Umsetzung gerührt. Die Aufarbeitung erfolgt bei dem erfindungsgemäßen Verfahren (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen Unkräutern in monokotylen und dikotylen Kulturen, vor allem bei verpflanztem Reis.

In gewissem Umfang zeigen die Verbindungen der Formel (I) auch insektizide, akarizide und fungizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflachenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfar stoffe und Spurennährstoffe wie Salze von Eisen, Mangan, Bor, Kupfer, Kobalt, Molybdhn und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl )-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

### Herstellungsbeispiele:

### Beispiel 1

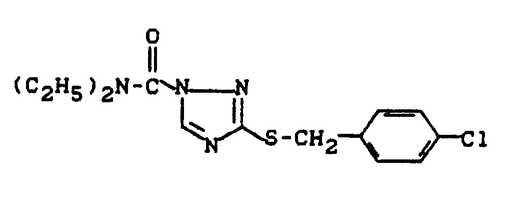

(Verfahren (a))

5,02 g (37 mMol) N,N-Diethylcarbamidsäurechlorid werden unter Rühren zu einer Mischung aus 8,0 g (35,5 mMol) 3-(4-Chlor-benzylthio)-2H-1,2,4-triazol und 50 ml Pyridin tropfenweise gegeben und das Gemisch wird 15 Stunden bei 20°C gerührt. Anschließend wird das Gemisch auf Wasser gegossen und mit Methylenchlorid extrahiert. Die organische Phase wird mit 2N-Salzsäure gewaschen, mit Natriumsulfat getrocknet und filtriert. Vom Filtrat wird das Lösungsmittel im Wasserstrahlvakuum sorgfältig abdestilliert.

Man erhält 9,8 g (85% der Theorie) 1-(Diethylaminocarbonyl)-3-(4-chlor-benzylthio)-1H-1,2,4-triazol als kristallinen Rückstand vom Schmelzpunkt 84°C-86°C.

### Beispiel 2

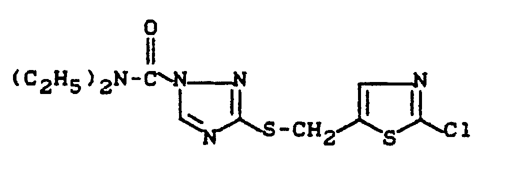

### (Verfahren (a))

3,5 g (26 mMol) N,N-Diethylcarbamidsäurechlorid werden unter Rühren zu einer Mischung aus 5,0 g (21,5 mMol) 3-(2-chlor-thiazol-5-yl-methylthio)-2H-1,2,4-triazol und 30 ml Pyridin tropfenweise gegeben und das Gemisch wird 15 Stunden bei 20°C gerührt. Anschließend wird das Gemisch auf Wasser gegossen und mit Methylenchlorid extrahiert. Die organische Phase wird mit 2H-Salzsäure gewaschen, mit Natriumsulfat getrocknet und filtriert. Vom Filtrat wird das Lösungsmittel im Wasserstrahlvakuum sorgfältig abdestilliert.

Man erhält 5,5 g (77% der Theorie) 1-(Diethylaminocarbonyl)-3-(2-chlor-thiazol-5-yl-methylthio)-1H-1,2,4-triazol als öligen Rückstand, der allmählich kristallisiert.

Schmelzpunkt: 55°C-58°C.

### Beispiel 3

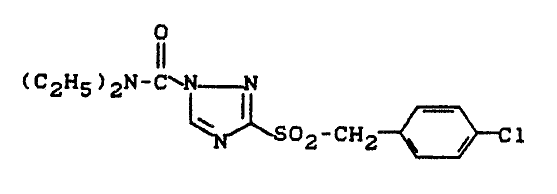

### (Verfahren (b))

Eine Mischung aus 6,6 g (20,3 mMol) 1-(Diethylaminocarbonyl)-3-(4-chlor-benzylthio)-1H-1,2,4-triazol - vgl. Bsp. 1 - und 100 ml Chloroform wird mit einem Eisbad gekühlt und portionsweise mit insgesamt 8,7 g (42,6 mMol) 3-Chlor-perbenzoesäure versetzt. Das Gemisch wird 15 Stunden bei 20°C gerührt und dann auf Wasser gegossen. Die organische Phase wird abgetrennt, mit Natriumsulfat getrocknet und filtriert. Vom Filtrat wird das Lösungsmittel im Wasserstrahlvakuum abdestilliert, der Rückstand mit Ethanol-Diethylether (Vol 1:1) verrührt und abgesaugt.

Man erhält 5,75 g (79,5% der Theorie) 1-(Diethylaminocarbonyl )-3-(4-chlor-phenylmethylsulfonyl)-1H-1,2,4-triazol vom Schmelzpunkt 117°C.

### Beispiel 4

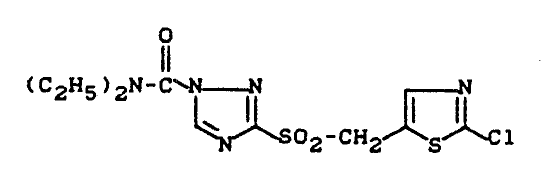

### (Verfahren (b))

Zu 3,8 g (11,5 mMol) 1-(Diethylaminocarbonyl)-3-(2-chlor-thiazol-5-yl-methylthio)-1H-1,2,4-triazol - vgl. Bsp. 2 - in 100 ml Chloroform werden unter Kühlen mit einem Eisbad 4,9 g (24,2 mMol) 3-Chlor-perbenzoesäure portionsweise gegeben. Das Gemisch wird 15 Stunden bei 20°C gerührt, dann mit gesättigter Natriumcarbonat-Lösung geschüttelt, mit Natriumsulfat getrocknet und filtriert. Vom Filtrat wird das Lösungsmittel im Wasserstrahlvakuum sorgfältig abdestilliert. Der Rückstand wird durch Säulenchromatographie (Kieselgel, Methylenchlorid) gereinigt.

Man erhält 3,0 g (72% der Theorie) 1-(Diethylaminocarbonyl)-3-(2-chlor-thiazol-5-yl-methylsulfonyl)-1H-1,2,4-triazol als kristallines Produkt vom Schmelzpunkt 122°C-124°C.

Analog zu den Beispielen 1 bis 4 sowie entsprechend den allgemeinen Angaben zur Durchführung der erfindungsgemäßen Verfahren können beispielsweise auch die in der nachstehenden Tabelle 1 aufgeführten Verbindungen der Formel (I) hergestellt werden.

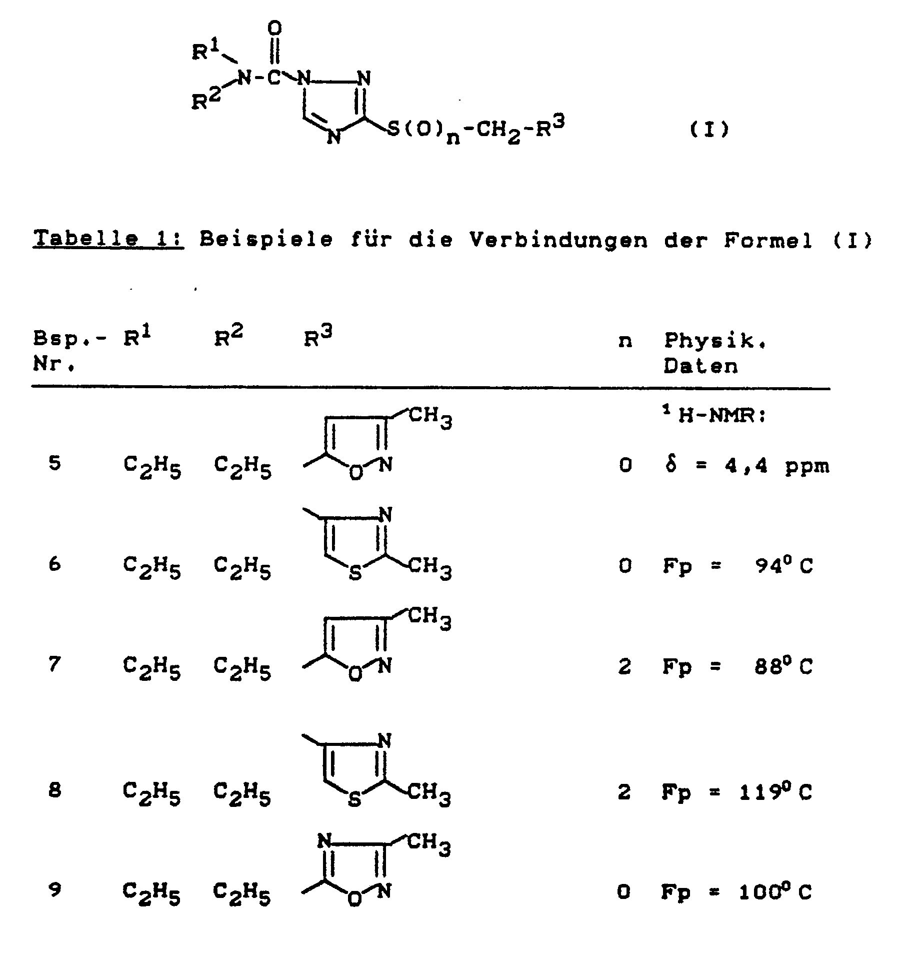

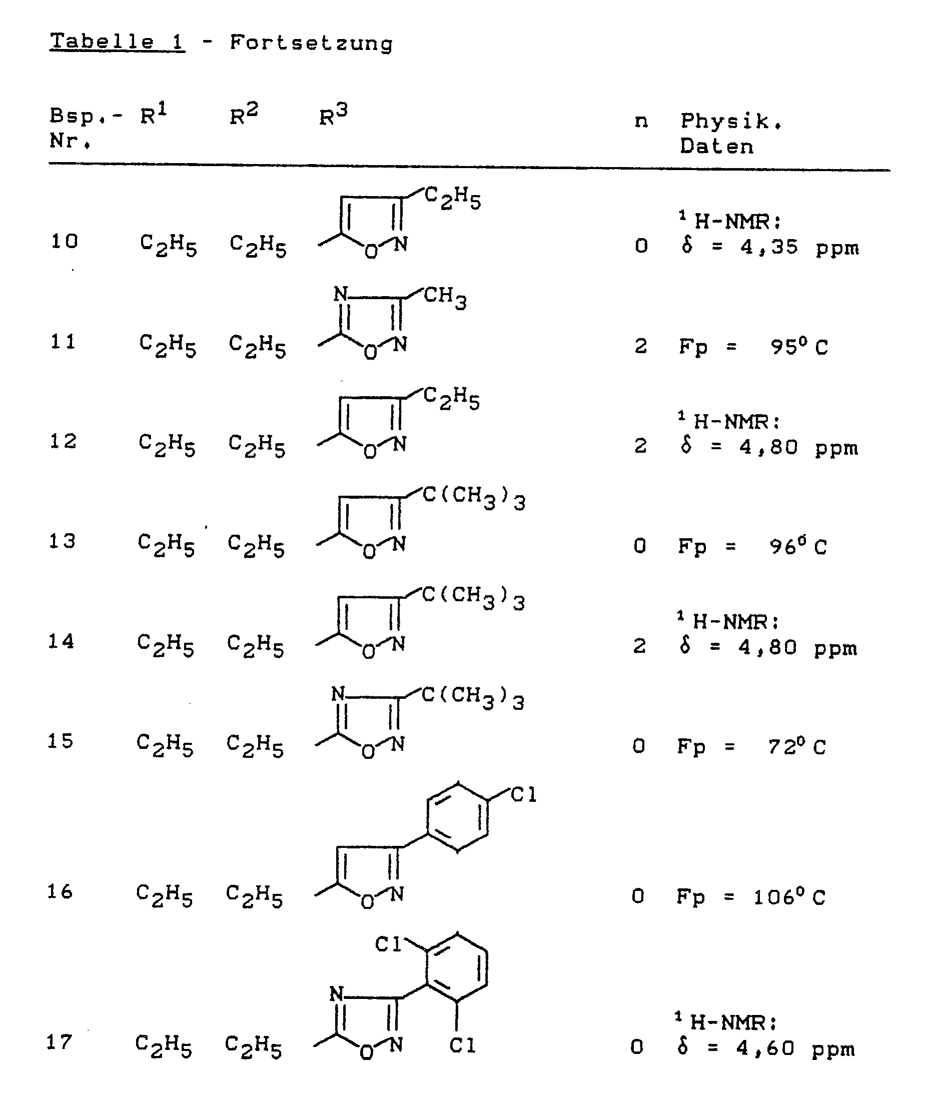

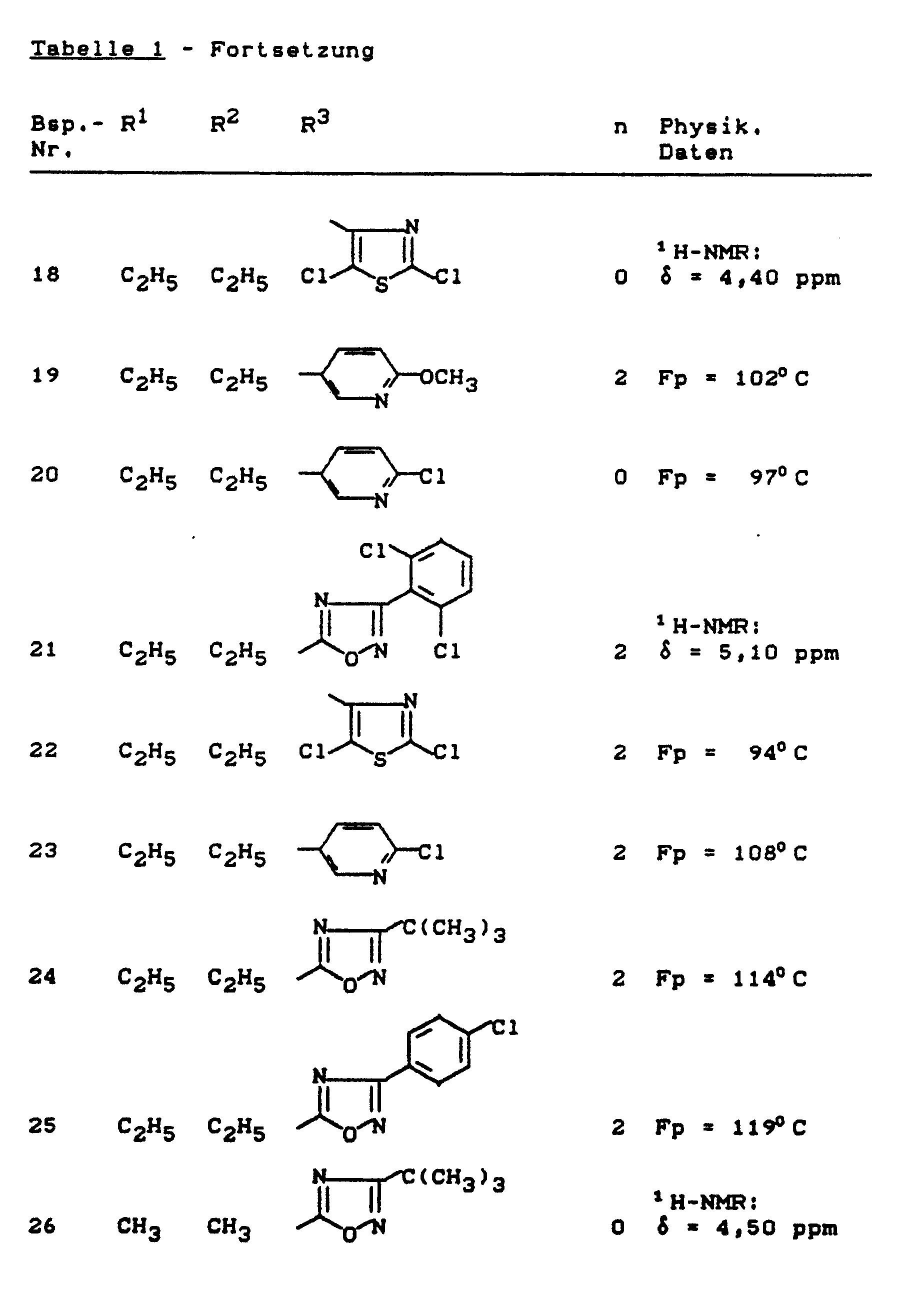

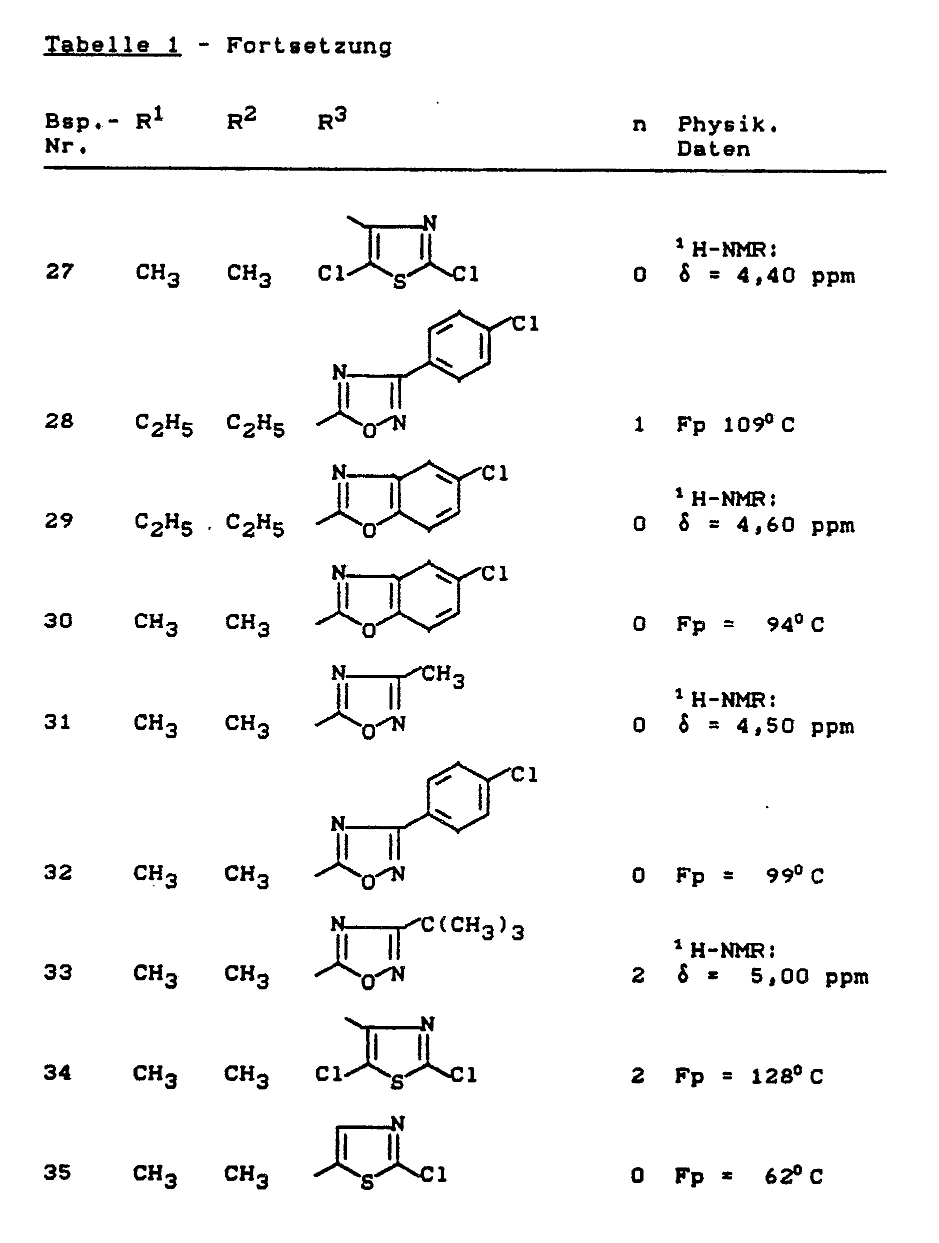

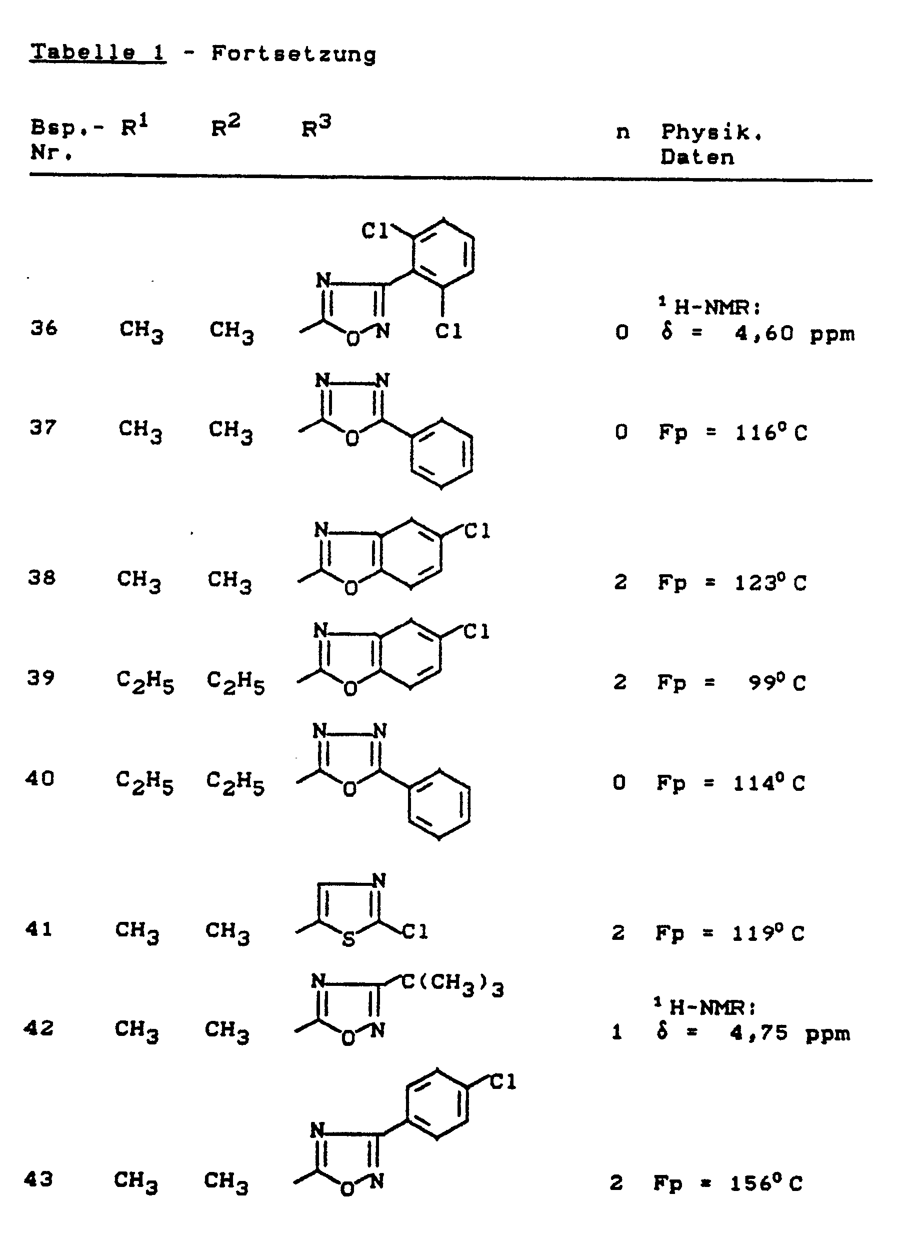

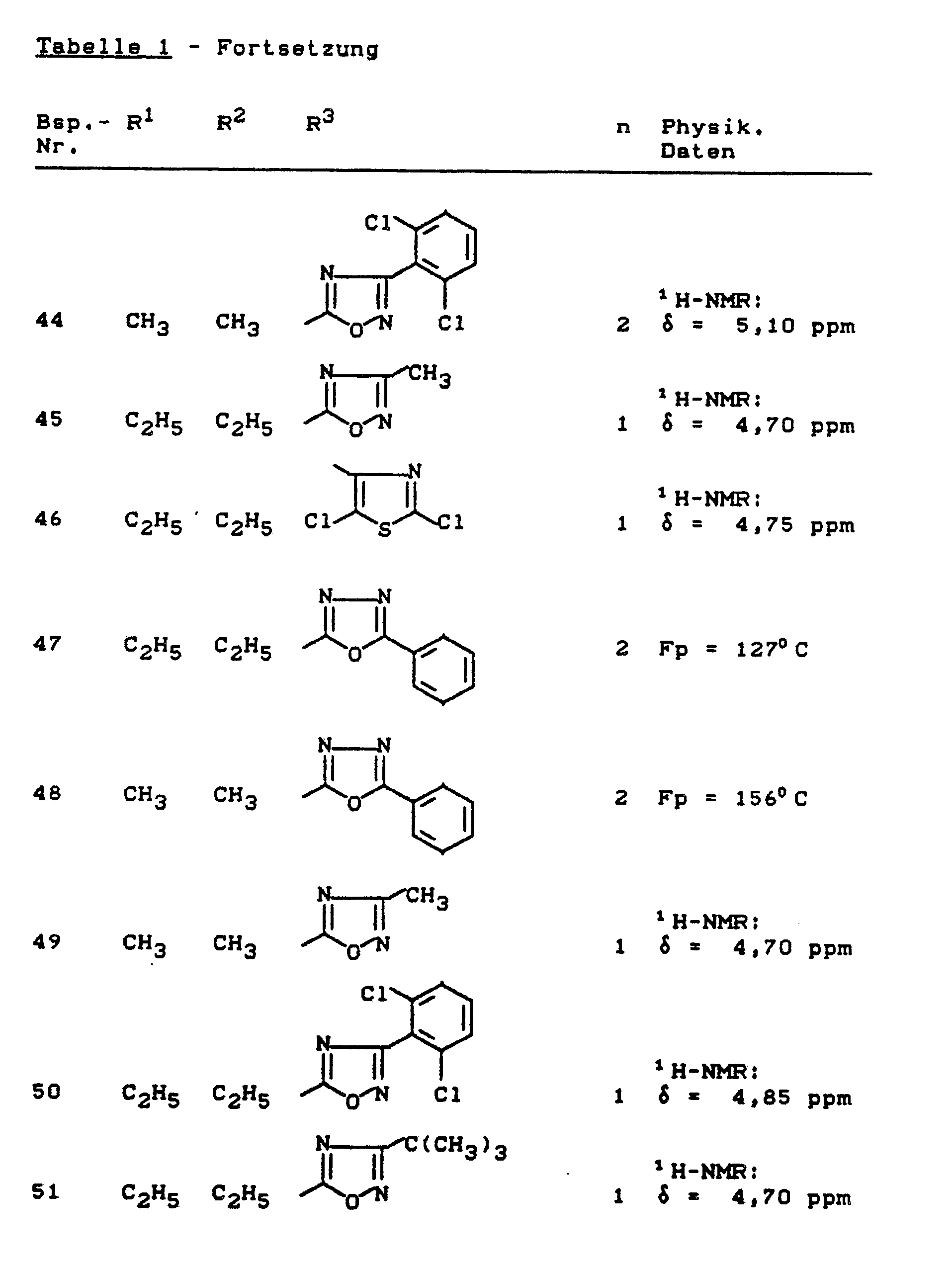

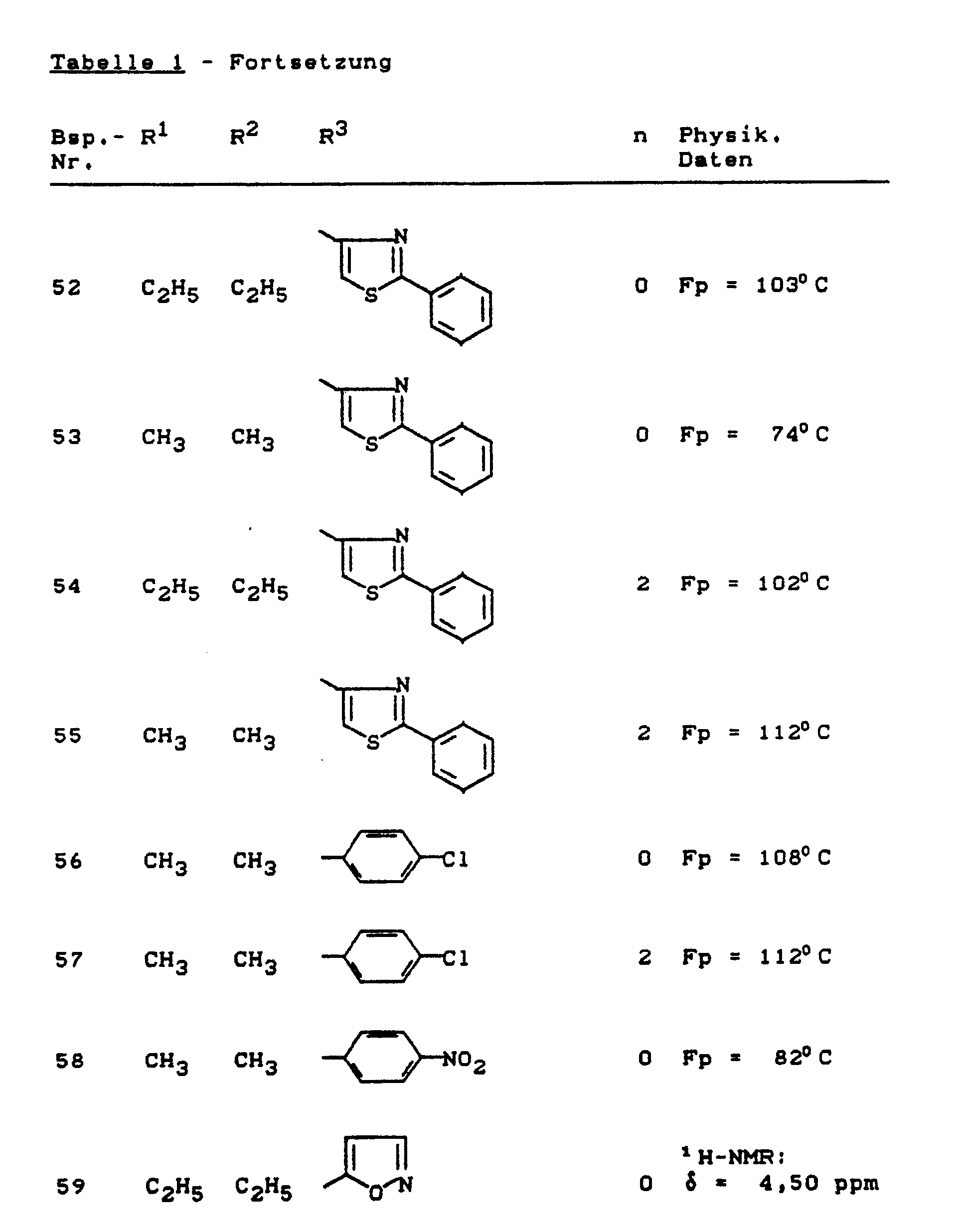

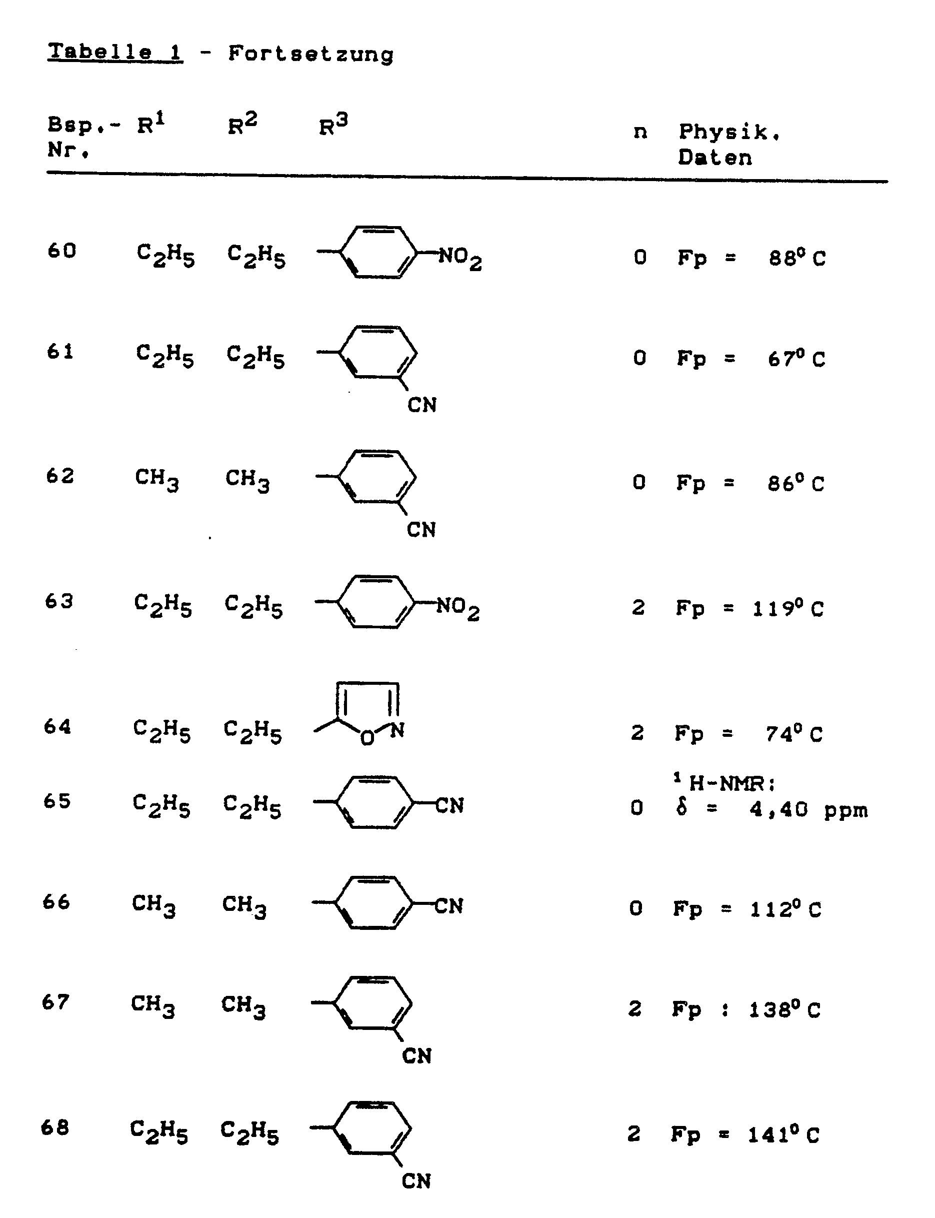

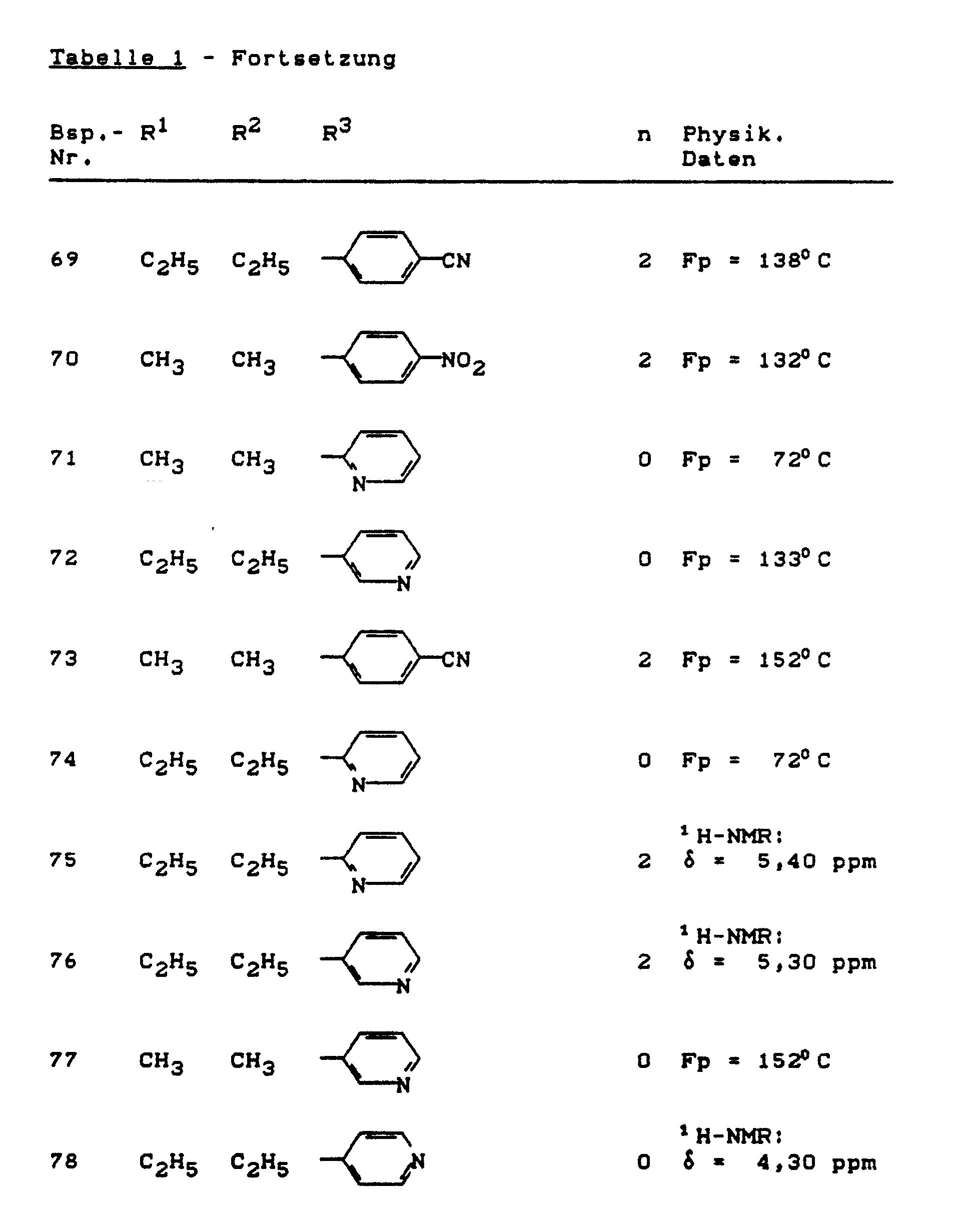

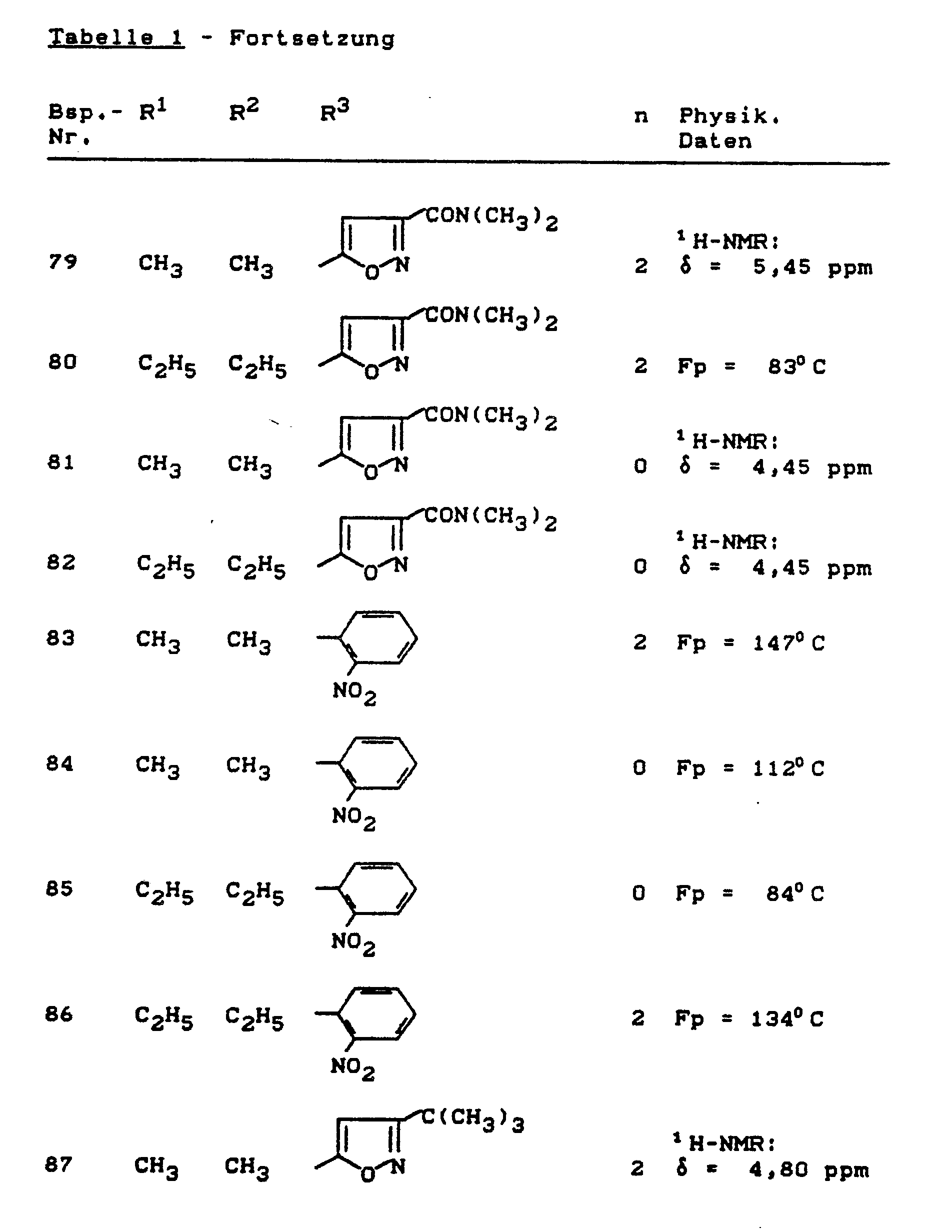

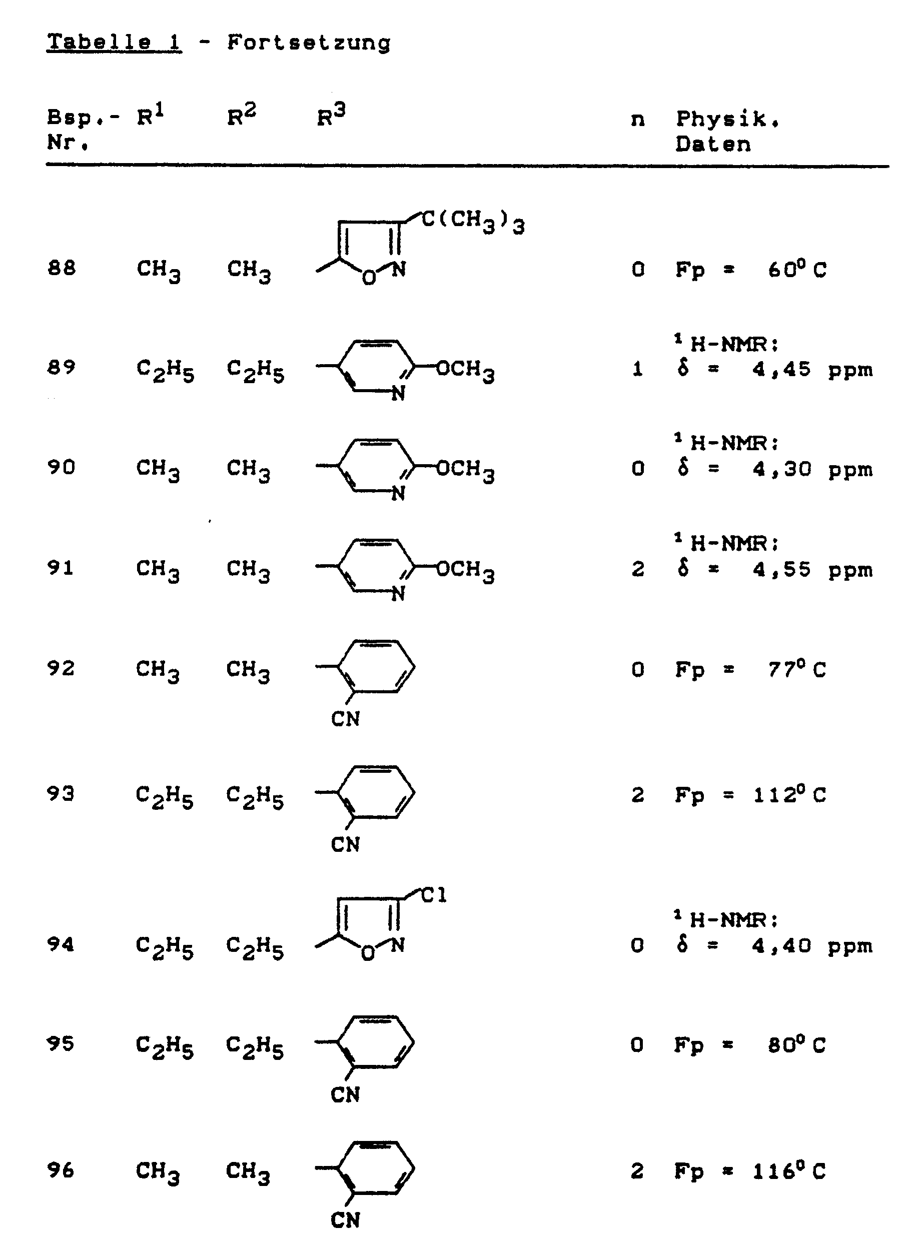

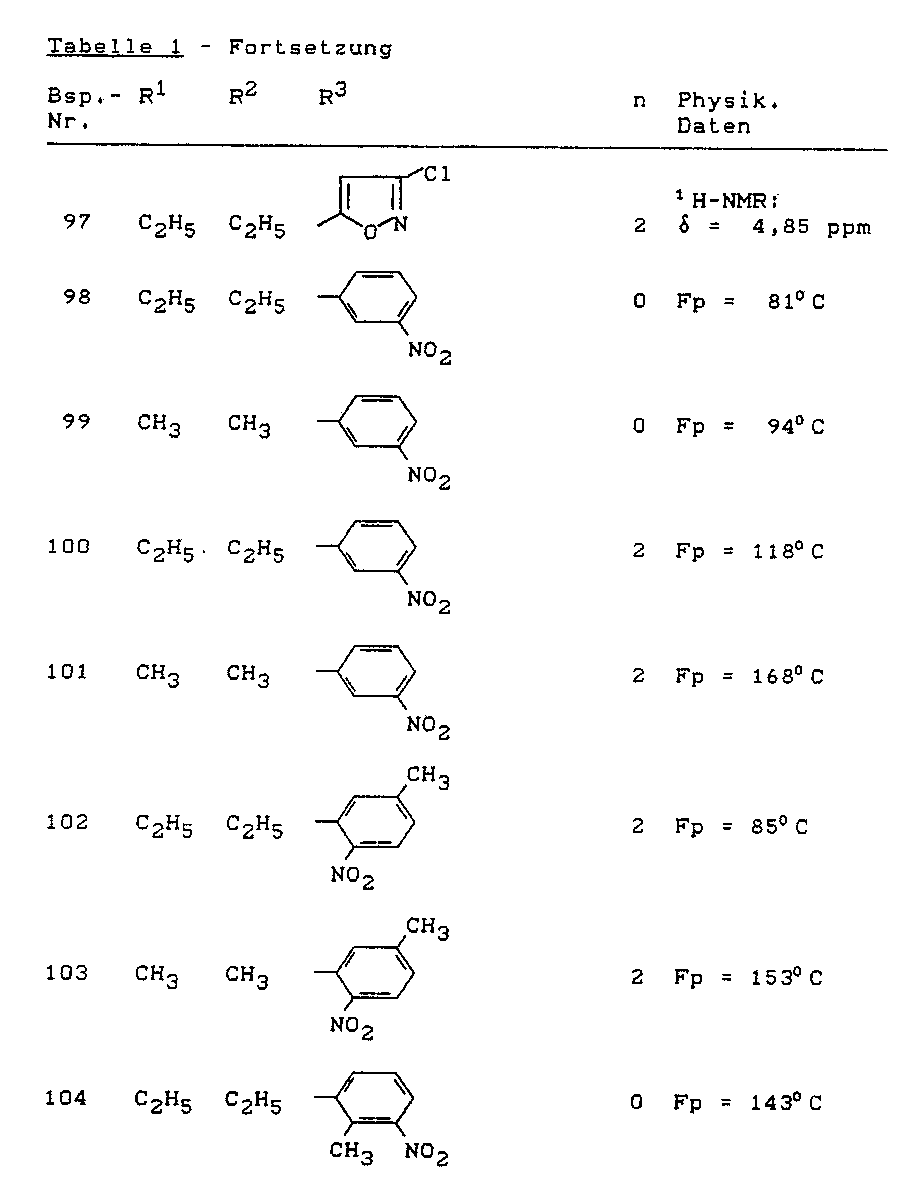

Soweit bei den physikalischen Daten δ-Werte angegeben sind beziehen sie sich auf die chemischen Verschiebungen in den ¹H-NMR-Spektren (aufgenommen in CDCl₃, Standard: Tetramethylsilan) für die in allen Verbindungen der Formel (I) vorhandene CH₂-Gruppierung.

### Ausgangsstoffe der Formel (II):

### Beispiel (II-1)

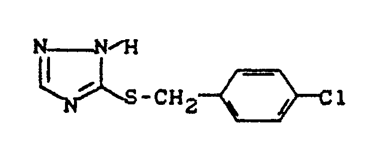

10,0 g (0,10 Mol) 3-Mercapto-1,2,4-triazol werden bei 20°C zu einer Lösung von 5,94 g (0,11 Mol) Natriummethanolat in 50 ml Methanol gegeben. Anschließend werden 15,8 g (0,098 Mol) 4-Chlor-benzylchlorid portionsweise dazugegeben und die Mischung wird 15 Stunden bei Raumtemperatur gerührt. Der Ansatz wird in Wasser gegossen und das hierbei kristallin angefallene Produkt durch Absaugen isoliert.

Man erhält 22,1 g (100% der Theorie) 3-(4-Chlor-benzylthio)-2H-1,2,4-triazol vom Schmelzpunkt 137°C-139°C.

### Beispiel (II-2)

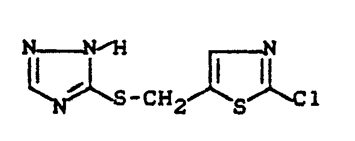

5,3 g (52 mMol) 3-Mercapto-1,2,4-triazol werden portionsweise zu einer Lösung von 2,81 g (52 mMol) Natriummethanolat in 20 ml Methanol gegeben. Dann wird eine Lö sung von 8,4 g (50 mMol) 2-Chlor-5-chlormethyl-thiazol in 20 ml Methanol zugetropft. Das Gemisch wird 15 Stunden bei 20°C gerührt und auf Wasser gegossen. Das hierbei kristallin angefallene Produkt wird durch Absaugen isoliert.

Man erhält 10,4 g (90% der Theorie) 3-(2-Chlor-thiazol-5-yl-methylthio)-2H-1,2,4-triazol vom Schmelzpunkt 121°C.

### Anwendungsbeispiele:

### Beispiel A

### Test an verpflanztem Wasserreis

Lösungsmittel: 5 Gewichtsteile Aceton
Emulgator : 1 Gewichtsteil Benzyloxypolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Pflanzgefäße (Oberfläche 1/5000 Ar) werden mit Boden aus einem Reisfeld gefüllt. Zwei Reispflanzen (Sorte: Kinmaze) werden im 2-3 Blattstadium (ca. 10 cm hoch) in die Gefäße verpflanzt. Samen von Echinochloa crus galli werden in die feucht gehaltene Erde ausgesät. 2 Tage nach dem Verpflanzen des Reises wird der Boden bis zu einer Wassertiefe von 3 cm überstaut. Die Wirkstoffzubereitung wird auf die Wasseroberfläche ausgebracht. Die Wirkstoffkonzentration in der Zubereitung spielt keine Rolle, entscheidend ist nur die Aufwandmenge des Wirkstoffs pro Flächeneinheit.

Nach der Anwendung des Wirkstoffs wird für 2 Tage durch die Pflanzgefäße ein vertikal absteigender Wasserstrom mit einer Geschwindigkeit von 2-3 cm pro Tag einge stellt. Danach werden die Testansätze unter Überflutungsbedingungen gehalten, wobei die Wassertiefe 3 cm beträgt.

Nach 4 Wochen wird der Schädigungsgrad der Pflanzen bonitiert in % Schädigung (bzw. Unkrautwirkung) im Vergleich zu einer unbehandelten Kontrolle.

Es bedeuten:

0 % = keine Wirkung (wie unbehandelte Kontrolle)
100 % = totale Vernichtung

In diesem Test zeigt sich die hervorragende Verträglichkeit der erfindungsgemäßen Wirkstoffe -insbesondere der Verbindung aus Beispiel 3 - in Reis bei gleichzeitig sehr guter Unkrautwirkung.

## Patentansprüche

1. Substituierte Carbamoyltriazole der allgemeinen Formel (I) 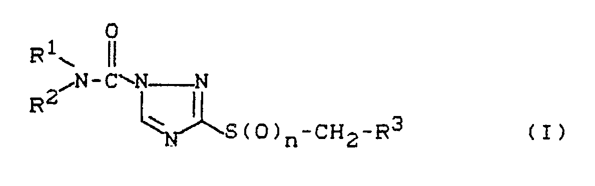 in welcher
R¹ und R² gleich oder verschieden sind und unabhängig voneinander jeweils für geradkettiges oder verzweigtes Alkyl mit 1 bis 6 Kohlenstoffatomen stehen,
R³ für 4-Chlor-phenyl, für Cyanophenyl, für Nitrophenyl, Methyl-nitrophenyl oder für einen fünfgliedrigen heteroaromatischen Cyclus steht, welcher ein oder zwei Stickstoffatome und zusätzlich ein Sauerstoff- oder Schwefelatom als Heteroatome enthält und welcher gegebenenfalls einfach oder zweifach, gleich oder verschieden durch Halogen, Cyano, Nitro, Dimethylaminocarbonyl, Diethylaminocarbonyl oder durch jeweils geradkettiges oder verzweigtes Alkyl, Alkoxy, Alkylthio, Alkylsulfinyl oder Alkylsulfonyl mit jeweils 1 bis 6 Kohlenstoffatomen oder durch Phenyl substituiert ist, wobei das Phenyl gegebenenfalls einfach bis dreifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ weiterhin für einen sechsgliedrigen heteroaromatischen Cyclus steht, welcher ein oder zwei Stickstoffatome enthält und welcher gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ ferner für Benzoxazolyl oder Benzthiazolyl steht, welche jeweils gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert sind, und
n für die Zahlen 0, 1 oder 2 steht.

2. Substituierte Carbamoyltriazole der Formel (I) gemäß Anspruch 1, in welcher
R¹ und R² gleich oder verschieden sind und unabhängig voneinander jeweils für geradkettiges oder verzweigtes Alkyl mit 1 bis 4 Kohlenstoffatomen, ausgenommen tert-Butyl, stehen,
R³ für 4-Chlor-phenyl, für Cyanophenyl, für Nitrophenyl, Methyl-nitrophenyl oder für einen fünfgliedrigen heteroaromatischen Cyclus aus der Reihe Oxazolyl, Isoxazolyl, Thiazolyl, Isothiazolyl, Oxadiazolyl, Thiadiazolyl steht, welcher gegebenenfalls einfach oder zweifach, gleich oder verschieden durch Fluor, Chlor, Brom, Dimethylaminocarbonyl oder durch jeweils geradkettiges oder verzweigtes Alkyl, Alkoxy, Alkylthio, Alkylsulfinyl oder Alkylsulfonyl mit jeweils 1 bis 4 Kohlenstoffatomen substituiert ist, oder welcher durch Phenyl substituiert ist, wobei das Phenyl gegebenenfalls einfach oder zweifach, gleich oder verschieden durch Fluor, Chlor, Brom, Methyl, Ethyl, Methoxy oder Ethoxy substituiert ist, oder R³ weiterhin für Pyridyl oder Pyrimidyl steht, welche gegebenenfalls einfach oder zweifach, gleich oder verschieden durch Fluor, Chlor, Brom, Methyl, Ethyl, Methoxy oder Ethoxy substituiert sind, oder R³ ferner für Benzoxazolyl oder Benzthiazolyl steht, welche jeweils gegebenenfalls einfach oder zweifach, gleich oder verschieden durch Fluor, Chlor, Brom, Methyl, Ethyl, Methoxy oder Ethoxy substituiert sind, und
n für die Zahlen 0, 1 oder 2 steht.

3. Substituierte Carbamoyltriazole der Formel (I) gemäß Anspruch 1, in welcher
R¹ für Methyl, Ethyl oder n-Propyl steht,
R² für Methyl, Ethyl, n-Propyl, Isopropyl, n-Butyl oder Isobutyl steht,
R³ für 4-Chlor-phenyl, für Cyanophenyl, für Nitrophenyl, für Isoxazolyl, welches gegebenenfalls durch Chlor, Methyl, Ethyl, Propyl, Isopropyl, Butyl, Isobutyl, sec-Butyl, tert-Butyl oder Dimethylaminocarbonyl substituiert ist, für Thiazolyl, welches gegebenenfalls durch Chlor, Methyl oder Phenyl substituiert ist, für Oxadiazolyl, welches gegebenenfalls durch Methyl, Ethyl, Propyl, Isopropyl, Butyl, Isobutyl, sec-Butyl, tert-Butyl, Phenyl, Chlorphenyl oder Dichlorphenyl substituiert ist, für Pyridyl, welches gegebenenfalls durch Chlor, Methyl oder Methoxy substituiert ist, oder für Benzoxazolyl, welches gegebenenfalls durch Chlor substituiert ist, steht, und
n für die Zahlen 0, 1 oder 2 steht.

4. Verbindung der Formel

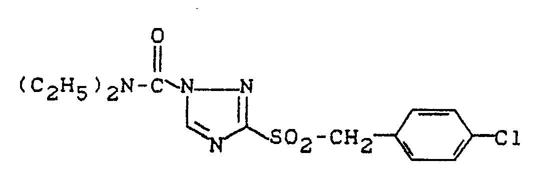

5. Verfahren zur Herstellung von substituierten Carbamoyltriazolen der Formel (I) 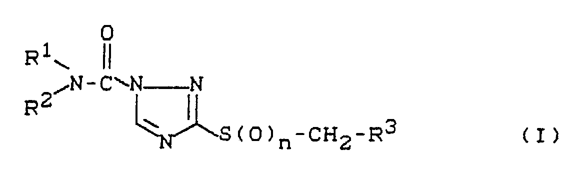 in welcher
R¹ und R² gleich oder verschieden sind und unabhängig voneinander jeweils für geradkettiges oder verzweigtes Alkyl mit 1 bis 6 Kohlenstoffatomen stehen,
R³ für 4-Chlor-phenyl, für Cyanophenyl, für Nitrophenyl, Methyl-nitrophenyl oder für einen fünfgliedrigen heteroaromatischen Cyclus steht, welcher ein oder zwei Stickstoffatome und zusätzlich ein Sauerstoff- oder Schwefelatom als Heteroatome enthält und welcher gegebenenfalls einfach oder zweifach, gleich oder verschieden durch Halogen, Cyano, Nitro, Dimethylaminocarbonyl, Diethylaminocarbonyl oder durch jeweils geradkettiges oder verzweigtes Alkyl, Alkoxy, Alkylthio, Alkylsulfinyl oder Alkylsulfonyl mit jeweils 1 bis 6 Kohlenstoffatomen oder durch Phenyl substituiert ist, wobei das Phenyl gegebenenfalls einfach bis dreifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ weiterhin für einen sechsgliedrigen heteroaromatischen Cyclus steht, welcher ein oder zwei Stickstoffatome enthält und welcher gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ ferner für Benzoxazolyl oder Benzthiazolyl steht, welche jeweils gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert sind, und
n für die Zahlen 0, 1 oder 2 steht,
dadurch gekennzeichnet, daß man
(a) substituierte Triazole der allgemeinen Formel (II) 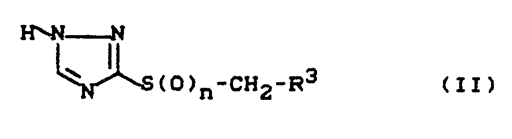 in welcher
R³ und n die oben angegebenen Bedeutungen haben,
oder Tautomere zu den Verbindungen der Formel (II)
mit Carbamidsäurechloriden der allgemeinen Formel (III) 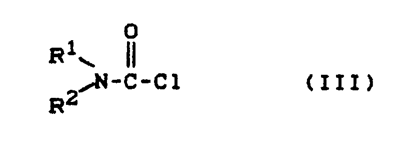 in welcher
R¹ und R² die oben angegebene Bedeutung haben,
gegebenenfalls in Gegenwart eines Säureakzeptors und gegebenenfalls in Gegenwart eines Verdünnungsmittels umsetzt, oder daß man
(b) substituierte Carbamoyltriazole der allgemeinen Formel (Ia) 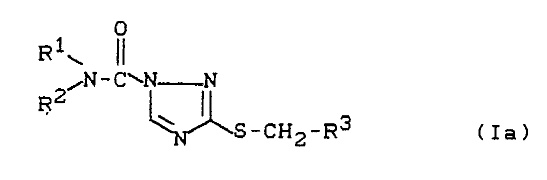 in welcher
R¹, R² und R³ die oben angegebene Bedeutung haben,
mit Oxidationsmitteln, gegebenenfalls in Gegenwart von Verdünnungsmitteln umsetzt.

6. Herbizide Mittel, gekennzeichnet durch einen Gehalt an mindestens einem substituierten Carbamoyltriazol der Formel (I) gemäß Anspruch 1, 4 oder 5.

7. Verfahren zur Bekämpfung von unerwünschten Pflanzen, dadurch gekennzeichnet, daß man substituierte Carbamoyltriazole der Formel (I) gemäß Anspruch 1, 4 oder 5 auf die Pflanzen und/oder ihren Lebensraum einwirken läßt.

8. Verwendung von substituierten Carbamoyltriazolen der Formel (I) gemäß Anspruch 1, 4 oder 5 zur Bekämpfung von unerwünschten Pflanzen.

9. Verfahren zur Herstellung von herbiziden Mitteln, dadurch gekennzeichnet, daß man substituierte Carbamoyltriazole der Formel (I) gemäß Anspruch 1, 4 oder 5 mit Streckmitteln und/oder oberflächenaktiven Substanzen vermischt.

10. Substituierte Triazole der allgemeinen Formel (II) 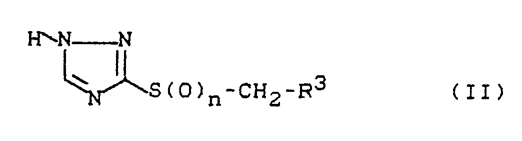 in welcher
R³ für 4-Chlor-phenyl, für Cyanophenyl, für Nitrophenyl, Methyl-nitrophenyl oder für einen fünfgliedrigen heteroaromatischen Cyclus steht, welcher ein oder zwei Stickstoffatome und zusätzlich ein Sauerstoff- oder Schwefelatom als Heteroatome enthält und welcher gegebenenfalls einfach oder zweifach, gleich oder verschieden durch Halogen, Cyano, Nitro, Dimethylaminocarbonyl, Diethylaminocarbonyl oder durch jeweils geradkettiges oder verzweigtes Alkyl, Alkoxy, Alkylthio, Alkylsulfinyl oder Alkylsulfonyl mit jeweils 1 bis 6 Kohlenstoffatomen oder durch Phenyl substituiert ist, wobei das Phenyl gegebenenfalls einfach bis dreifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ weiterhin für einen sechsgliedrigen heteroaromatischen Cyclus steht, welcher ein oder zwei Stickstoffatome enthält und welcher gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert ist, oder R³ ferner für Benzoxazolyl oder Benzthiazolyl steht, welche jeweils gegebenenfalls einfach oder zweifach, gleich oder verschieden durch Halogen, Cyano, Nitro oder durch jeweils geradkettiges oder verzweigtes Alkyl oder Alkoxy mit jeweils 1 bis 4 Kohlenstoffatomen substituiert sind, und
n für die Zahlen 0, 1 oder 2 steht,
oder deren Tautomere.
